# EUROPEAN PATENT APPLICATION

(11) **EP 4 797 269 A1**
(43) Date of publication of application: **26.08.2026**
(21) Application number: 24897977.5
(22) Date of filing: 18.11.2024
(51) Int. Cl.: G16C 20/70, G16C 20/30, G06N 20/00

(54) **PHYSICAL PROPERTY DATA ACQUISITION DEVICE AND PHYSICAL PROPERTY DATA ACQUISITION METHOD**

(30) Priority: 29.11.2023 KR 20230169806
(71) Applicant: LG Chem, Ltd., Seoul 07336 (KR)
(72) Inventor: KIM, Hyeong Seok, Daejeon 34122 (KR); OH, Soo Seok, Daejeon 34122 (KR); AHN, Deuk Hwan, Daejeon 34122 (KR); PARK, Yeon Jae, Daejeon 34122 (KR); KIM, Jun Young, Daejeon 34122 (KR); PARK, Si Young, Daejeon 34122 (KR)
(74) Representative: Goddar, Heinz J.
(86) International application number: PCT/KR2024/018138
(87) International publication number: WO 2025/116381

(57) **Abstract**

Disclosed herein is an apparatus for acquiring material property data, including a communication unit that receives a plurality of numerical measurement data, and a control unit that generates preprocessing data by adding an index column to the plurality of numerical measurement data, inputs the preprocessing data into a regression model for deriving material property data, and acquires the material property data as an output value based on hyperparameters set in the regression model.

## Description

### Technical Field

### CROSS-REFERENCE TO RELATED APPLICATION

This application claims priority to and the benefit of Korean Patent Application No. 10-2023-0169806 filed in the Korean Intellectual Property Office on November 29, 2023, the entire content of which is incorporated herein by reference.

### TECHNICAL FIELD

The embodiments disclosed in this document are related to an apparatus and method for acquiring material property data.

### Background Art

Recently, plastic is being used in various fields such as automobiles and electronic products, and in order for product manufacturers to design products using plastic, various material property information needs to be considered.

Accordingly, in the related art, material properties of the materials were evaluated through various testing equipment. However, there were difficulties in acquiring high-quality material property data due to the noise generated by the testing equipment and the cumulative deviations from repeated experiments. Therefore, the acquisition of high-quality material property data through machine learning techniques is necessary.

### Disclosure

### Technical Problem

According to an embodiment disclosed in this document, an apparatus and method for acquiring material property data is provided, which derives representative material property information by inputting measurement data into a training model.

Technical problems of the embodiments disclosed in the present document are not limited to the aforementioned technical problems, and other technical problems, which are not mentioned above, may be clearly understood by those skilled in the art from the following descriptions.

### Technical Solution

There is provided an apparatus for acquiring material property data, according to an embodiment. The apparatus may include: a communication unit configured to receive a plurality of numerical measurement data and a control unit configured to generate preprocessing data by adding an index column to the plurality of numerical measurement data, input the preprocessing data into a regression model for deriving material property data, and acquire the material property data as an output value based on hyperparameters set in the regression model.

The control unit may generate the preprocessing data by pairing the index column and the plurality of measurement data corresponding the index column on a one-to-one basis, for each of the plurality of measurement data.

The control unit may generate a representative material property curve based on the material property data and transmit the representative material property curve to an external device through the communication unit.

The control unit may repeatedly derive the material property data and merge the material property data through data-based merging to derive the representative material property curve.

The regression model may be configured by combining a linear regression model and a Gaussian process regression model.

The control unit may, based on receiving a user command to adjust the hyperparameters through the communication unit, adjust parameters of a radial basis function (RBF) kernel included in the Gaussian process regression model to acquire the material property data.

The control unit may add an index column to Poisson ratio data included in the plurality of numerical measurement data to generate preprocessing data, and acquire the material property data based on the preprocessing data.

There is provided a method of acquiring material property data, according to an embodiment. The method may include: receiving a plurality of numerical measurement data; generating preprocessing data by adding an index column to the plurality of numerical measurement data; inputting the preprocessing data into a regression model for deriving material property data; and acquiring the material property data as an output value based on hyperparameters set in the regression model.

The generating of the preprocessing data may include generating the preprocessing data by pairing the index column and the plurality of measurement data corresponding the index column on a one-to-one basis, for each of the plurality of measurement data.

The method may further include: generating a representative material property curve based on the material property data; and transmitting the representative material property curve to an external device through the communication unit.

The generating of the representative material property curve includes: deriving repeatedly the material property data; and merging the material property data through data-based merging to derive the representative material property curve.

The regression model may be configured by combining a linear regression model and a Gaussian process regression model.

The method of acquiring material property data, according to an embodiment may further include: acquiring the material property data by adjusting parameters of a radial basis function (RBF) kernel included in the Gaussian process regression model, based on receiving a user command to adjust the hyperparameters through the communication unit.

The acquiring of the material property data may include: adding an index column to Poisson ratio data included in the plurality of numerical measurement data to generate preprocessing data; and acquiring the material property data based on the preprocessing data.

### Advantageous Effects

According to the apparatus for acquiring material property data according to an embodiment, high-quality material property data can be acquired by filtering the noise of the material property data through machine learning techniques.

### Description of Drawings

FIG. 1 illustrates a control block diagram of an apparatus for acquiring material property data according to an embodiment.
FIG. 2 illustrates the input and output values of the machine learning model used in the apparatus for acquiring material property data, according to an embodiment.
FIG. 3 illustrates a schematic flowchart in which the apparatus for acquiring material property data generates material property data, according to an embodiment.
FIG. 4 illustrates the preprocessing data generated by the apparatus for acquiring material property data according to an embodiment.
FIG. 5 illustrates the measurement data used in the apparatus for acquiring material property data according to an embodiment.
FIG. 6 illustrates the result of the measurement data being matched with an index by the apparatus for acquiring material property data according to an embodiment.
FIG. 7 illustrates the noise filtering performance according to hyperparameters in the apparatus for acquiring material property data according to an embodiment.
FIGS. 8 to 10 illustrate the representative material property curves acquired by the apparatus for acquiring material property data according to an embodiment.
FIG. 11 illustrates a control flowchart of a method of acquiring material property data according to an embodiment.

### Mode for Disclosure

Hereinafter, various embodiments disclosed in the present document will be described in detail with reference to the accompanying drawings. In the drawings of the present document, the same constituent elements will be designated by the same reference numerals, and the repetitive description of the same constituent elements will be omitted.

Specific structural or functional descriptions of various embodiments disclosed in the present document are exemplified only for the purpose of explaining the embodiments, the embodiments disclosed in the present document may be carried out in various forms, and it should not be interpreted that the present document is limited to the embodiments described in the present document.

The terms "first" and "second" used in various embodiments may be used regardless of the order and/or importance of various constituent elements and do not limit the corresponding constituent elements. For example, a first constituent element may be named a second constituent element, and similarly, the second constituent element may also be named the first constituent element, without departing from the scope of the embodiment disclosed in the present document.

The terms used in the present document are used to just describe a specific embodiment and do not intend to limit the scope of another embodiment. Singular expressions may include plural expressions unless clearly described as different meanings in the context.

All terms used herein, including technical or scientific terms, may have the same meaning as commonly understood by those skilled in the art to which the embodiments disclosed in the present document pertain. The terms such as those defined in a commonly used dictionary may be interpreted as having meanings identical or similar to meanings in the context of related technologies and should not be interpreted as ideal or excessively formal meanings unless explicitly defined in the present document. In some instances, the terms defined in the present document should not be interpreted to exclude the embodiments disclosed in the present document.

Hereinafter, the principles of operation and embodiment of the disclosure will be described in detail with reference to the accompanying drawings.

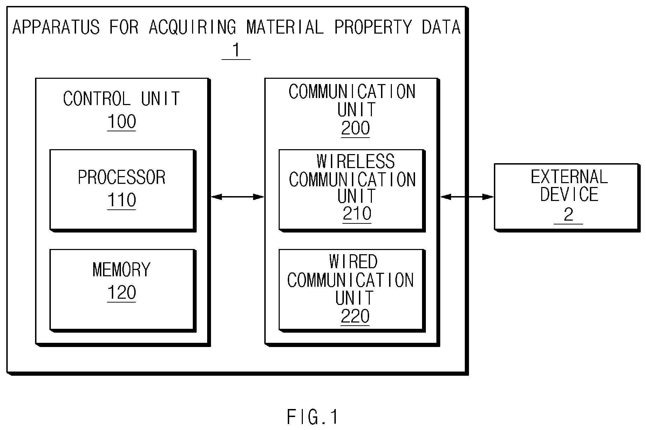

FIG. 1 illustrates a control block diagram of an apparatus for acquiring material property data according to an embodiment.

With reference to FIG. 1, an apparatus 1 for acquiring material property data according to an embodiment includes a control unit 100 including at least one processor 110 and memory 120, as well as a communication unit 200, which communicates with an external device 2 through the communication unit 200 to transmit material property data to the outside.

The apparatus 1 for acquiring material property data according to an embodiment may be implemented as a server that acquires material property data based on machine learning, and more specifically, may be implemented as various computing devices such as a workstation, cloud, data drive, or data station. In addition, the apparatus 1 for acquiring material property data may be implemented as one or more server devices physically or logically separated based on functions, detailed configurations of the functions, or data, and may transmit and receive data through communication between the respective server devices and process the transmitted and received data.

The external device 2 that communicates with the apparatus 1 for acquiring material property data may include a user terminal and an external server device that receive the material property data generated by the apparatus 1 for acquiring material property data.

Specifically, when the external device 2 is a user terminal, the control unit 100 of the apparatus 1 for acquiring material property data may transmit the material property data generation result to the user terminal so that the user may check the result. In this case, the user terminal may include a personal computer, terminal, portable telephone, smartphone, handheld device, wearable device, and the like, but is not limited thereto.

Additionally, when the external device 2 is an external server device, the external server device may be implemented as various computing devices, and the external server device may be implemented as one or more external server devices. Through communication between the respective external server devices, data may be transmitted and received, and the transmitted and received data may be processed.

The apparatus 1 for acquiring material property data according to an embodiment may refer to any electronic device that includes the processor 110 and the memory 120. Hereinafter, the constituent elements of the apparatus 1 for acquiring material property data will be described in detail.

The communication unit 200 may include a wireless communication unit 210 and a wired communication unit 220 to communicate with the external device 2. The communication unit 200 may receive numerical measurement data from the separately provided external device 2 to be used as training data, or transmit and receive programs and various data for machine learning.

The wireless communication unit 210 may include at least one of a short-range communication module or a long-range communication module.

The short-range communication module may communicate with the adjacent external device 2 to the apparatus 1 for acquiring material property data using a short-range communication method. Here, the short-range communication module may use one of the communication methods such as Bluetooth, Bluetooth Low Energy, Infrared Data Association (IrDA), Zigbee, Wi-Fi, Wi-Fi Direct, Ultra Wideband (UWB), or Near Field Communication (NFC).

The long-range communication module may include a communication module that performs various types of long-range communication and may include a mobile communication unit. The mobile communication unit may transmit and receive wireless signals with at least one of a base station, an external terminal, or the external device 2 on a mobile communication network. Additionally, the long-range communication module may communicate with external devices 2, such as the external device 2 or another electronic device, through a nearby access point (AP). The access point (AP) may connect the local area network (LAN) to which the apparatus 1 for acquiring material property data is connected to a wide area network (WAN) connected to a communication server. Accordingly, the apparatus 1 for acquiring material property data may be connected to a communication server through the external device 2 and the wide area network (WAN), allowing them to communicate with each other.

The wired communication unit 220 may connect to a wired communication network and communicate with the external device 2 through the wired communication network. For example, the wired communication unit 220 may connect to a wired communication network via Ethernet (IEEE 802.3 technology standard) or connect to a wired communication network via controller area network (CAN) communication, and transmit and receive data with external devices 2 through the wired communication network.

The apparatus 1 for acquiring material property data according to an embodiment may include an input/output interface (not illustrated). The apparatus 1 for acquiring material property data may provide an interface that connects input devices (not illustrated) such as a keyboard, mouse, or touch panel, and output devices such as a display (not illustrated) with the processor 110 to transmit and receive data.

The memory 120 may store various information necessary for the operation of the apparatus 1 for acquiring material property data. Specifically, the memory 120 may store the operating system and programs necessary for the operation of the apparatus 1 for acquiring material property data, or store data required for the operation of the apparatus 1 for acquiring material property data.

Specifically, the memory 120 may store various programs related to measurement data preprocessing, representative material property curve derivation algorithms, and machine learning algorithms. Additionally, the memory 120 may store feature data used in machine learning.

The memory 120 may include volatile memory 120 such as static random access memory (S-RAM) or dynamic random access memory (D-RAM) for temporarily storing data. In addition, the memory 120 may include non-volatile memory 120 such as read-only memory (ROM), erasable programmable read-only memory (EPROM), or electrically erasable programmable read-only memory (EEPROM) for long-term data storage.

The processor 110 outputs control signals to overall control the apparatus 1 for acquiring material property data. The processor 110 may include one or a plurality of central processing units (CPU) and graphics processing units (GPU). In this case, the processor 110 may be implemented as an array of multiple logic gates, or it may be implemented as a combination of a general-purpose microprocessor 110 and the memory 120 in which programs executable on the microprocessor 110 are stored.

The aforementioned memory 120 and processor 110 may be included in the control unit 100, and the control unit 100 may acquire high-quality material property data by controlling the aforementioned constituent elements.

Specifically, the control unit 100 may generate preprocessing data by adding an index column to a plurality of numerical measurement data. That is, the control unit 100 may preprocess the measurement data to ensure that the data is effectively learned and filtered in the machine learning model, and may separate the measurement data representing a plurality of features by adding an index column.

That is, the control unit 100 may generate the preprocessing data by pairing the index column and the plurality of measurement data corresponding the index column on a one-to-one basis, for each of the plurality of measurement data.

Subsequently, the control unit 100 may input the preprocessing data into a regression model and acquire the material property data as an output value based on the hyperparameters set in the regression model. In this case, the hyperparameters refer to variables that are adjusted by the learning algorithm of the machine learning model itself. The hyperparameters are set before the learning of the model and may be used to control and adjust the learning process of the model.

The control unit 100 may generate a representative material property curve based on the material property data and transmit the representative material property curve to the external device 2 through the communication unit 200. Here, the representative material property curve refers to a curve that represents a specific material or material property, and may include curves such as the tensile stress-strain curve and torque-z displacement curve.

Additionally, the control unit 100 may repeatedly derive material property data and merge the material property data through data-based merging to derive the representative material property curve, thereby acquiring material property data with high versatility and consistency.

In addition, based on receiving a user command to adjust the hyperparameters through the communication unit 200, the control unit 100 may adjust the parameters of the radial basis function (RBF) kernel included in the Gaussian process regression model to acquire the material property data.

That is, the user may adjust the hyperparameters of the machine learning model, thereby adjusting the noise filtering and data merging performance.

Accordingly, since the apparatus 1 for acquiring material property data may acquire high-quality material property data using a machine learning model, it has the effect of reducing the time and cost required for the process of acquiring material property data.

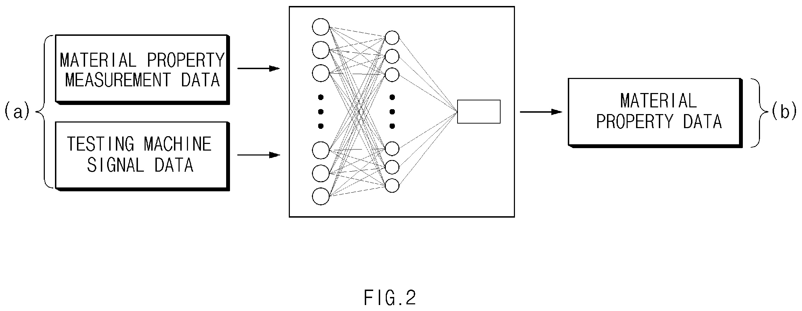

FIG. 2 illustrates the input and output values of the machine learning model used in the apparatus 1 for acquiring material property data, according to an embodiment.

The control unit 100 may use the material property measurement data included in a plurality of numerical measurement images and the signal data from a testing machine as training data (a) to train the machine learning model, and derive the representative material property data as the output data (b).

When the control unit 100 includes the processor 110 (e.g., an NPU) dedicated to artificial intelligence for training the machine learning model, the processor 110 may use the weight data stored in the memory 120 as training data for the machine learning model to train the artificial neural network.

Examples of learning algorithms include supervised learning, unsupervised learning, semi-supervised learning, and reinforcement learning, but are not limited to the aforementioned examples.

The artificial neural network included in the machine learning model may be composed of a plurality of neural network layers. Each of the plurality of neural network layers has a plurality of weights and performs neural network calculations through calculations between the results of the previous layer's calculations and the plurality of weights. The plurality of weights that the plurality of neural network layers have may be optimized through the learning results of the artificial intelligence model. For example, during the learning process, the plurality of weights may be updated so that the loss value or cost value acquired by the artificial intelligence model decreases or is minimized.

The artificial neural network may include a deep neural network (DNN), and examples include convolutional neural networks (CNN), deep neural networks (DNN), recurrent neural networks (RNN), restricted Boltzmann machines (RBM), deep belief networks (DBN), bidirectional recurrent deep neural networks (BRDNN), deep Q-networks (Deep Q-Networks), or others, but are not limited to the aforementioned examples.

The control unit 100 may learn the correlation between the material property measurement data included in the numerical measurement data, the testing machine signal data, and the representative material property data based on the selected artificial intelligence model.

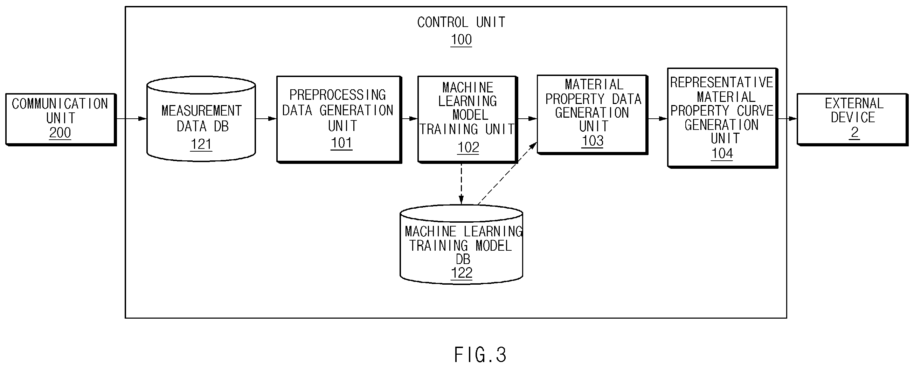

FIG. 3 illustrates a schematic flowchart in which the apparatus 1 for acquiring material property data generates material property data, according to an embodiment.

The configurations 101 to 105 in FIG. 3 are implemented in the form of software blocks and are stored in the memory 120, where they may be executed by the processor 110.

First, with reference to FIG. 3, the control unit 100 may receive a plurality of numerical measurement data through the communication unit 200 and store the received measurement data in a measurement data DB 121. In this case, the plurality of numerical measurement data may include measurements used to measure the material properties of a material, such as tensile strength, tensile strain, hardness, toughness, thermal conductivity, and coefficient of thermal expansion.

The preprocessing data generation unit 101 of the control unit 100 may perform data preprocessing to process the received measurement data into training data. Data preprocessing refers to the process of converting raw measurement data into a form that may be applied to a machine learning model, and it may include all processes aimed at cleaning the data and ensuring that the model may easily learn patterns.

Specifically, the control unit 100 may transform the numerical measurement data to preprocess the data by replacing x-values into indices and mapping y-values to the existing data on a one-to-one basis. Additionally, the control unit 100 may preprocess the data by removing missing values and strings from the numerical measurement data.

Subsequently, the machine learning model training unit 102 of the control unit 100 may train the model based on the preset hyperparameters and selected features. Specifically, the control unit 100 may learn the parameters of the model using the selected features as input data to generate material property data by filtering the measurement data.

The apparatus 1 for acquiring material property data according to an embodiment may use an algorithm that combines the linear regression model and the Gaussian process regression model to acquire high-quality material property data.

Specifically, the control unit 100 may combine the linear regression model and the Gaussian process regression model, leveraging the flexibility of the Gaussian process and the simplicity and interpretability of the linear regression model to improve the performance of the model.

In this case, the linear regression model refers to an algorithm that models the linear relationship between a dependent variable and one or more independent variables, aiming to find a single line that best describes the data. Specifically, the linear regression model is used to predict continuous values for given input variables, and typically, methods such as the least squares method may be used to estimate the model parameters.

Additionally, the Gaussian process regression model is a probabilistic model that uses a Gaussian process to model the relationship between given data points, and may predict a probability distribution for a given input, thereby modeling the uncertainty in the data. The Gaussian process regression model may estimate the relationship between data points using a kernel function that depends on the training data.

Subsequently, the control unit 100 may store the trained machine learning model in a machine learning training model DB 122.

The material property data generation unit 103 of the control unit 100 may generate material property data corresponding to the output value of the training model. Specifically, the control unit 100 may use the smooth curve derivation characteristics of the radial basis function (RBF) kernel in the Gaussian process regression model, and regardless of the form of the numerical measurement data which is an input value, it may perform noise filtering and data merging to generate material property data.

Subsequently, the representative material property curve generation unit 104 of the control unit 100 may generate the representative material property curve through the merging of repeated experiment data. Specifically, the control unit 100 performs a data-based merging rather than a formula-based merging, which provides the advantage of easier specialization and consistency across different fields.

The control unit 100 may generate the representative material property curve and transmit it to the external device 2, thereby efficiently providing the material property of the corresponding material to users who wish to use the material property data.

As described above, the apparatus 1 for acquiring material property data according to an embodiment preprocesses numerical measurement data by applying machine learning and generates material property data based on preset hyperparameters, thereby enabling the acquisition of reliable material property data. As a result, the acquisition of material property data becomes easier compared to conventional methods, and the quality of material property data, which cannot be achieved by conventional technologies, may be secured.

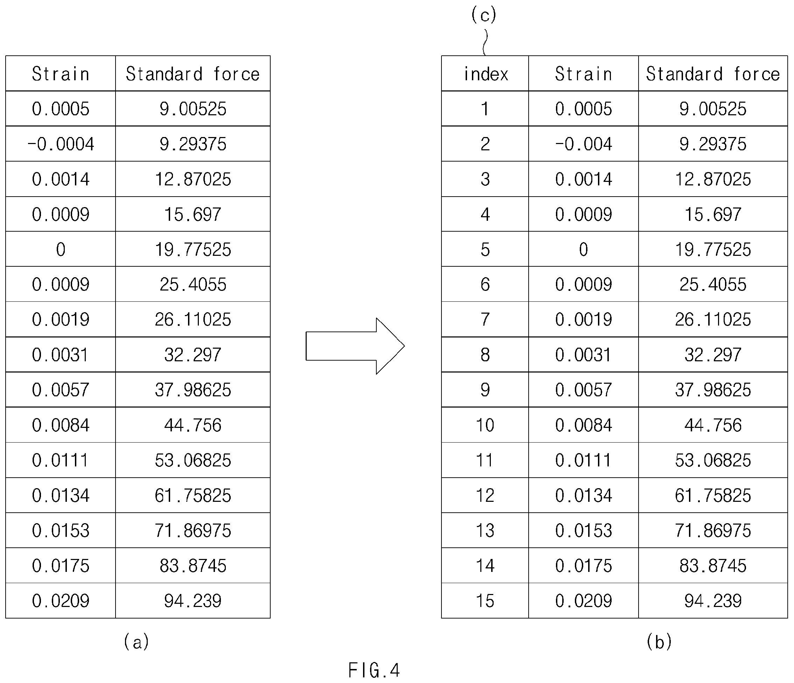

FIG. 4 illustrates the preprocessing data generated by the apparatus 1 for acquiring material property data according to an embodiment.

With reference to FIG. 4, the control unit 100 may receive strain-standard force data as measurement data, where the strain-standard force data refers to a set of data points related to the strain and the applied force obtained from a material's tensile test.

Here, (a) in FIG. 4 refers to the raw measurement data received through the communication unit 200, and (b) in FIG. 4 refers to the preprocessing data that has been processed by the control unit 100.

Generally, strain-standard force data is represented as pairs and is visualized in a graph for use in describing material properties. Accordingly, the raw measurement data (a) received through the communication unit 200 may consist of pairs of strain data and applied force data.

However, the apparatus 1 for acquiring material property data according to an embodiment may acquire preprocessing data (b) by matching each data characteristic included in the raw measurement data (a) to a unified index for effective filtering and machine learning training.

For example, in (a) of FIG. 4, the strain values are sequentially formed as 0.0005, - 0.0004, 0.0014, and 0.0009 from the top, and may be matched to the corresponding applied force (standard force) values of 9.00525, 9.29375, 12.87025, and 15.697, respectively, upon being received.

The control unit 100 may add an index column (c) to each characteristic value, where, for the strain values, 0.0005 is matched to index 1, -0.0004 is matched to index 2, 0.0014 is matched to index 3, and 0.0009 is matched to index 4. Additionally, the control unit 100 may, for the applied force (standard force) values, match 9.00525 to index 1, 9.29375 to index 2, 12.87025 to index 3, and 15.697 to index 4, from the top.

Accordingly, based on the index, information on the measurement data points may be easily derived, which may improve the performance of data analysis and modeling.

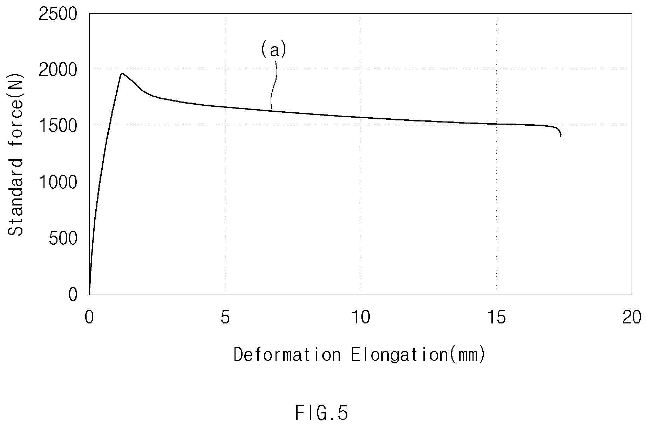

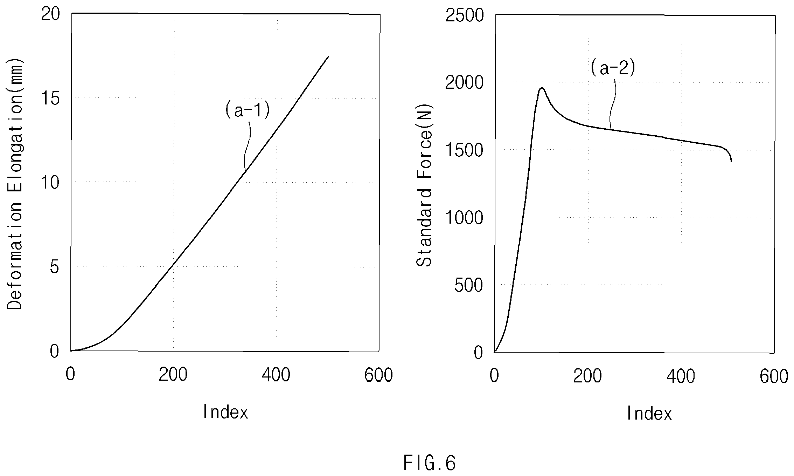

FIG. 5 illustrates the measurement data used in the apparatus 1 for acquiring material property data according to an embodiment, and FIG. 6 illustrates the result of the measurement data being matched with an index by the apparatus 1 for acquiring material property data according to an embodiment.

First, (a) in FIG. 5 represents the relationship between the force (standard force) applied to the material and the deformation elongation, which may refer to a graph visually representing the data obtained from a tensile test of the material.

Here, (a) in FIG. 5 initially shows a linear region where the deformation increases linearly, and after a certain point, the deformation may either remain constant or slightly decrease. However, when (a) is input into the training model without preprocessing, noise may affect the correlation of each data point with the output value.

Continuing to refer to FIG. 6, the material property data generation device according to an embodiment may match each characteristic value to a unified index and perform learning while minimizing noise.

That is, the control unit 100 may match the data related to the deformation elongation characteristic, as shown in (a-1) in FIG. 6, to indices of 0 to 600, thereby enabling the learning of the deformation elongation characteristic.

Similarly, the control unit 100 may match the data related to the applied force (standard force) characteristic, as shown in (a-2) in FIG. 6, to indices of 0 to 600, thereby enabling the learning of the applied force characteristic.

Subsequently, the control unit 100 may learn each characteristic matched to the index using a machine learning algorithm, then merge the data related to each characteristic. Based on the merged data, the control unit 100 may derive the representative material property curve.

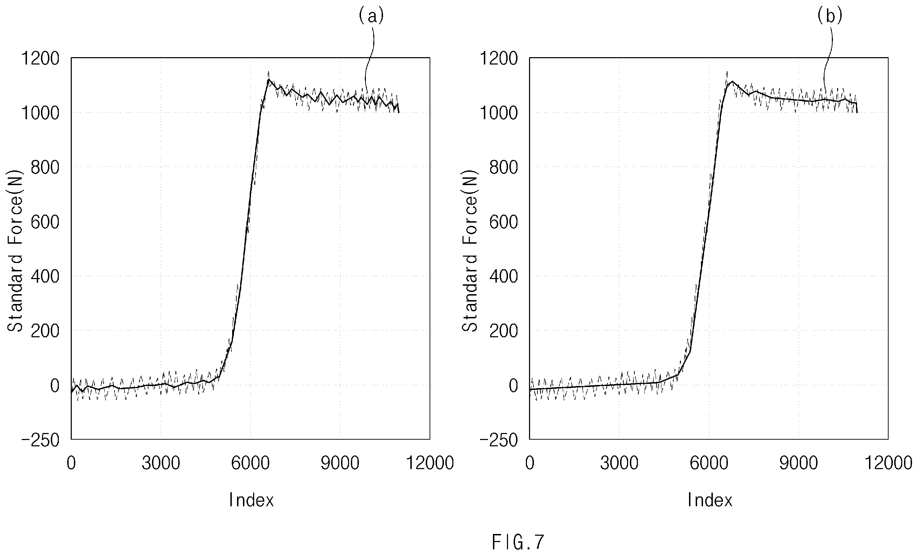

FIG. 7 illustrates the noise filtering performance according to hyperparameters in the apparatus 1 for acquiring material property data according to an embodiment.

The apparatus 1 for acquiring material property data according to an embodiment may adjust the degree of noise filtering in the material property data based on hyperparameters input by the user. Accordingly, since the user may intuitively set the hyperparameters based on the index, the overall performance of noise filtering and data merging may be improved.

Specifically, the graph in FIG. 7 shows the noise filtering performance according to hyperparameters when learning is performed on high-speed tensile test (high speed tensile) data. The graph represents the magnitude of the force (standard force) applied to the material according to the index.

Here, (a) in FIG. 7 may refer to the case where a specific hyperparameter is set to 10 for the high-speed tensile test data, and (b) in FIG. 7 may refer to the case where the same hyperparameter is set to 1000, as in (a). In this case, the value of the hyperparameter is illustrative and may vary depending on the training model and training data.

With reference to (a), the centerline may appear thicker due to noise compared to (b), where the noise has been reduced, and the centerline may appear thinner than in (a).

In this manner, the apparatus 1 for acquiring material property data according to an embodiment may receive hyperparameters from the user and adjust the performance of noise filtering based on the hyperparameters, thereby enabling the derivation of optimal material property data.

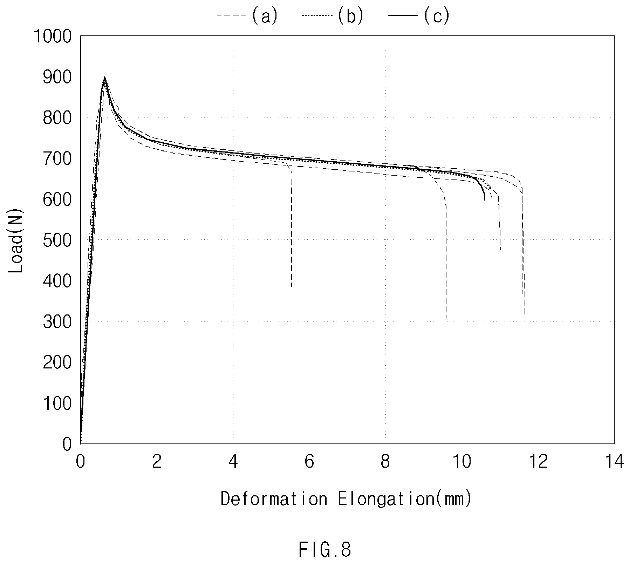

FIGS. 8 to 10 illustrate the representative material property curves acquired by the apparatus 1 for acquiring material property data according to an embodiment.

The control unit 100 may generate the representative material property curve for each material based on the material property data derived through machine learning. Here, the representative material property curve may refer to a representative graph that illustrates the physical, chemical, and mechanical properties of a specific material.

Specifically, FIG. 8 refers to the representative material property curves derived in different ways based on the stress (Load) and deformation elongation data acquired from a tensile test.

Here, (a) in FIG. 8 corresponds to raw measurement data, which includes multiple noises and may result in an uneven representative material property curve being acquired.

Additionally, (b) in FIG. 8 may refer to the representative material property curve acquired using a formula-based data merging method, while (c) in FIG. 8 may refer to the graph of the representative material property curve acquired using a data-based data merging method by the control unit 100.

Here, (b) in FIG. 8 and (c) in FIG. 8 show trends that may be similar, as the material property data were acquired using machine learning techniques. However, since the graph of (c) merges the data based on the data, it has the effect of superior versatility compared to the graph of (b).

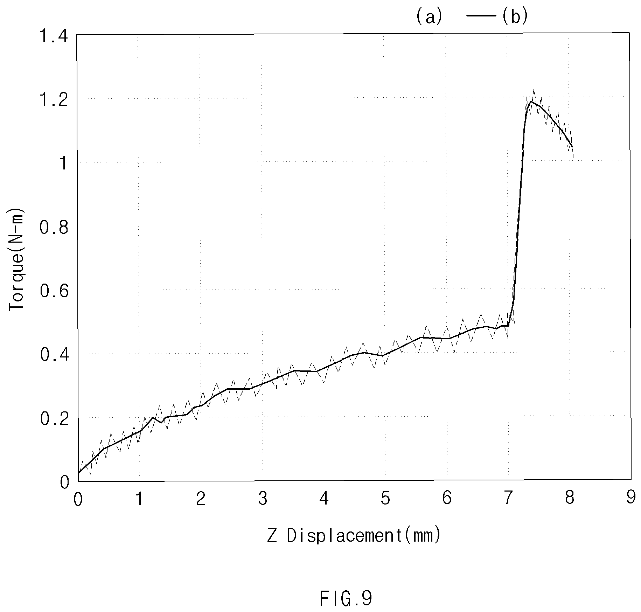

Next, FIG. 9 refers to the representative material property curve derived by the control unit 100 based on the torque-z displacement data acquired from the tensile test.

Here, (a) in FIG. 9 corresponds to raw measurement data, which includes multiple noises and may result in an uneven representative material property curve being acquired.

Meanwhile, (b) in FIG. 9 may refer to the graph of the representative material property curve acquired using a data-based data merging method by the control unit 100.

Comparing (a) and (b) in FIG. 9, (b) in FIG. 9 shows the material property data acquired using machine learning techniques. Compared to the raw measurement data, the linear regions are more prevalent, making it easier to identify trends. This allows for easier understanding and prediction of the material's characteristics through the trends.

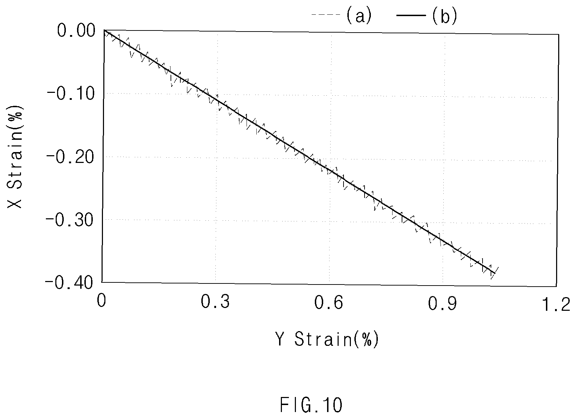

Next, FIG. 10 refers to the representative material property curve for Poisson ratio derived based on the X-strain-Y-strain (strain-strain) data acquired from the tensile test.

Here, (a) in FIG. 10 corresponds to raw measurement data, which includes multiple noises and may result in an uneven representative material property curve being acquired.

Meanwhile, (b) in FIG. 10 may refer to the graph of the representative material property curve acquired using a data-based data merging method by the control unit 100.

Comparing (a) and (b) in FIG. 10, (b) in FIG. 10 shows the material property data acquired using machine learning techniques. Compared to the raw measurement data, it may be expressed in linear regions, making it easier to identify the material's characteristics.

As a result, the apparatus 1 for acquiring material property data according to an embodiment acquires preprocessing data by matching the representative material property curve to the index, and merges the result of inputting the preprocessing data into the machine learning model based on data. This allows for the acquisition of material property data with minimal noise influence and high versatility.

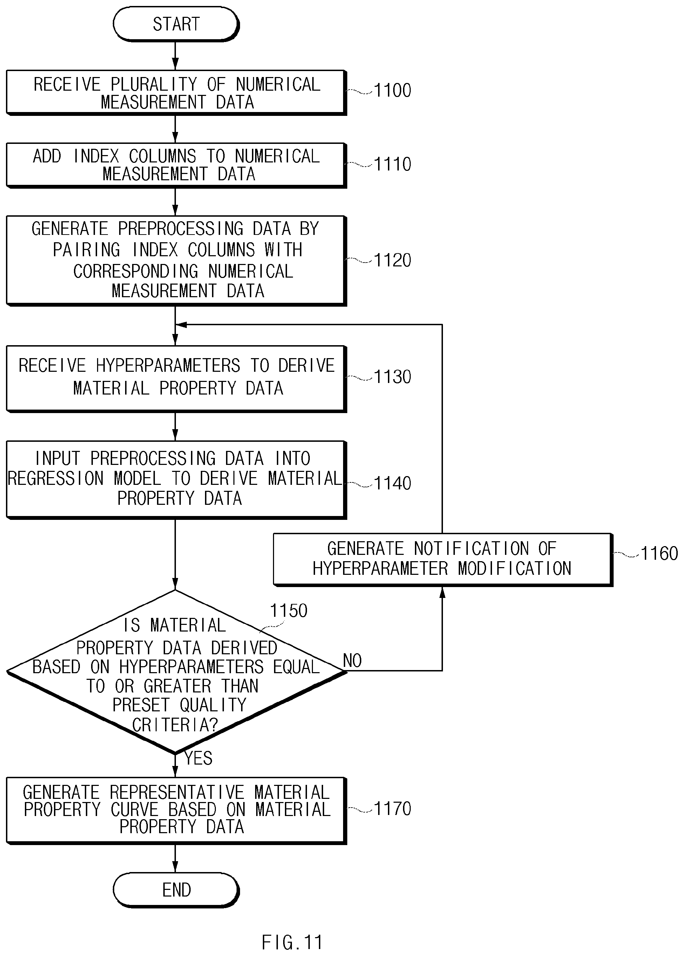

FIG. 11 illustrates a control flowchart of a method of acquiring material property data according to an embodiment.

With reference to FIG. 11, the control unit 100 may receive a plurality of numerical measurement data through the communication unit 200 (1100). Subsequently, the control unit 100 may add an index column to the received measurement data (1110), and specifically, pair the measurement data with the corresponding index column to generate preprocessing data (1120). That is, the control unit 100 may separate the measurement data expressed as data pairs and generate preprocessing data as index-data pairs.

Subsequently, the control unit 100 may receive hyperparameters for deriving material property data (1130). In this case, the hyperparameters may refer to values preset by the user, and based on the hyperparameters, the training of the machine learning model may be performed.

The control unit 100 may input the preprocessing data into a regression model for deriving material property data (1140), and based on the hyperparameters, the control unit 100 may determine whether the derived material property data is equal to or greater than preset quality criteria (1150). Specifically, a reference value indicating whether the material property data sufficiently reflects the characteristics of the material may be stored in the memory 120, and the control unit 100 may compare the material property data with the reference value to determine whether the material property data is equal to or greater than the quality criteria.

When the material property data derived based on the hyperparameters is determined to be below the preset quality criteria (NO in 1150), the control unit 100 may generate a notification requesting the modification of the hyperparameters (1160). However, the notification requesting the modification of the hyperparameters may be omitted, and the modification may be made directly by the user.

Subsequently, when the material property data derived based on the hyperparameters is determined to be equal to or greater than the preset quality criteria (YES in 1150), the control unit 100 may generate the representative material property curve based on the material property data (1170), and transmit the representative material property curve to the external device 2 through the communication unit 200.

As described above, according to the apparatus 1 for acquiring material property data in an embodiment, machine learning-based regression techniques may be used to acquire material property data, resulting in the advantage of generating material property data with clearer trends.

On the other hand, the disclosed embodiments may be implemented in the form of a recording medium that stores computer-executable instructions. The instruction may be stored in the form of a program code. When the instruction is executed by a processor, a program module may be generated, and operations of the disclosed embodiments may be performed. The recording medium may be implemented as a computer-readable recording medium.

Examples of the computer-readable recording medium include all kinds of recording media for storing instructions readable by a computer. Examples of the computer-readable recording medium may include a read-only memory (ROM), a random-access memory (RAM), a magnetic tape, a magnetic disc, a flash memory, an optical data storage device, or the like.

Additionally, the computer-readable recording medium may be provided in the form of a non-transitory storage medium. Here, the term "non-transitory storage medium" refers to a tangible device that does not include a signal (e.g., electromagnetic waves), and this term does not distinguish between cases where data is stored semi-permanently on the storage medium or temporarily stored. For example, a "non-transitory storage medium" may include a buffer where data is temporarily stored.

According to an embodiment, the methods according to various embodiments disclosed in this document may be included in and provided as a computer program product. The computer program product may be traded as a product between a seller and a buyer. The computer program product may be distributed in the form of a machine-readable recording medium (e.g., compact disc read only memory (CD-ROM)), or be distributed (e.g., downloaded or uploaded) online via an application store (e.g., PLAYSTORETM), or between two user devices (e.g., smart phones) directly. In case of online distribution, at least a portion of the computer program product (e.g., a downloadable app) may be at least temporarily stored or temporarily generated on a machine-readable recording medium, such as the memory of the manufacturer's server, the application store's server, or a relay server.

The above describes and illustrates specific embodiments. However, the invention is not limited to the specific embodiments described above, and a person skilled in the art to which the invention pertains would be able to make various modifications without departing from the scope of the technical spirit of the invention as set forth in the following claims.

### [Description of Reference Numerals]

1: Apparatus for acquiring material property data
2: External device
100: Control unit
110: Processor
120: Memory
121: Measurement data DB
122: Machine learning training model DB
200: Communication unit
210: Wireless communication unit
220: Wired communication unit

## Claims

1. An apparatus for acquiring material property data, comprising:
a communication unit configured to receive a plurality of numerical measurement data; and
a control unit configured to generate preprocessing data by adding an index column to the plurality of numerical measurement data, input the preprocessing data into a regression model for deriving material property data, and acquire the material property data as an output value based on hyperparameters set in the regression model.

2. The apparatus of claim 1, wherein the control unit generates the preprocessing data by pairing the index column and the plurality of measurement data corresponding the index column on a one-to-one basis, for each of the plurality of measurement data.

3. The apparatus of claim 1, wherein the control unit generates a representative material property curve based on the material property data and transmits the representative material property curve to an external device through the communication unit.

4. The apparatus of claim 3, wherein the control unit repeatedly derives the material property data and merges the material property data through data-based merging to derive the representative material property curve.

5. The apparatus of claim 1, wherein the regression model is configured by combining a linear regression model and a Gaussian process regression model.

6. The apparatus of claim 5, wherein the control unit, based on receiving a user command to adjust the hyperparameters through the communication unit, adjusts parameters of a radial basis function (RBF) kernel included in the Gaussian process regression model to acquire the material property data.

7. The apparatus of claim 1, wherein the control unit adds an index column to Poisson ratio data included in the plurality of numerical measurement data to generate preprocessing data, and acquires the material property data based on the preprocessing data.

8. A method of acquiring material property data, comprising:
receiving a plurality of numerical measurement data;
generating preprocessing data by adding an index column to the plurality of numerical measurement data;
inputting the preprocessing data into a regression model for deriving material property data; and
acquiring the material property data as an output value based on hyperparameters set in the regression model.

9. The method of claim 8, wherein the generating of the preprocessing data includes generating the preprocessing data by pairing the index column and the plurality of measurement data corresponding the index column on a one-to-one basis, for each of the plurality of measurement data.

10. The method of claim 8, further comprising:
generating a representative material property curve based on the material property data; and
transmitting the representative material property curve to an external device through the communication unit.

11. The method of claim 10, wherein the generating of the representative material property curve includes:
deriving repeatedly the material property data; and
merging the material property data through data-based merging to derive the representative material property curve.

12. The method of claim 8, wherein the regression model is configured by combining a linear regression model and a Gaussian process regression model.

13. The method of claim 12, further comprising:
acquiring the material property data by adjusting parameters of a radial basis function (RBF) kernel included in the Gaussian process regression model, based on receiving a user command to adjust the hyperparameters through the communication unit.

14. The method of claim 8, wherein the acquiring of the material property data includes:
adding an index column to Poisson ratio data included in the plurality of numerical measurement data to generate preprocessing data; and
acquiring the material property data based on the preprocessing data.
